# EUROPEAN PATENT SPECIFICATION

(11) **EP 4 429 747 B1**
(45) Date of publication and mention of the grant of the patent: **02.09.2026**
(21) Application number: 23747487.9
(22) Date of filing: 20.01.2023
(51) Int. Cl.: A61M 25/06, A61M 25/00

(54) **INTRAVENOUS CATHETER ASSEMBLIES INCLUDING CLOSED SYSTEM CATHETER ASSEMBLIES AND NEEDLE INSERTION ASSEMBLIES THEREFOR**
INTRAVENÖSE KATHETERANORDNUNGEN MIT KATHETERANORDNUNGEN MIT GESCHLOSSENEM SYSTEM UND NADELEINFÜHRUNGSANORDNUNGEN DAFÜR
ENSEMBLES CATHÉTERS INTRAVEINEUX COMPRENANT DES ENSEMBLES CATHÉTERS À SYSTÈME FERMÉ ET ENSEMBLES D'INSERTION D'AIGUILLE AFFÉRENTS

(30) Priority: 27.01.2022 US 202263303745 P
(43) Date of publication of application: 18.09.2024
(73) Proprietor: ICU Medical, Inc., San Clemente, CA 92673 (US)
(72) Inventor: ROEHL, Christopher, D., Plymouth, MN 55442 (US); KOEHLER, Thomas, T., Plymouth, MN 55442 (US)
(74) Representative: Venner Shipley LLP
(86) International application number: PCT/US2023/011191
(87) International publication number: WO 2023/146794

(56) References cited:
- EP-B1- 2 618 885
- WO-A1-2019/199736
- JP-A- 2021 130 048
- US-A1- 2008 147 003
- US-A1- 2008 147 003
- US-A1- 2018 289 932
- US-A1- 2019 388 653
- US-A1- 2019 388 653
- US-B2- 10 543 343
- US-B2- 7 972 313

## Description

### Field of the Invention

The present invention relates to safety intravenous catheter assemblies including closed system catheter (CSC) assemblies having a catheter hub with a side port, and needle insertion assemblies connectable to the catheter hub.

### Background of the Invention

In US 10,675,440, assigned to the same assignee as the instant application, a closed system catheter (CSC) assembly is described to have a catheter insertion device coupled to a catheter hub assembly that has a catheter hub with a side opening having connected thereto an extension tubing. The catheter insertion device has a needle housing that has at its distal end a passive release mechanism for connecting to the proximal end of the catheter hub. The passive release mechanism comprises an actuator internally movable relative to an output collar attached to the needle housing.
Other prior art arrangements are taught in WO 2019/199736, US 10543343, EP 2618885, US 2019/388653 and US 2008/147003.

### Summary of the Present Invention

The needle insertion assembly of the instant invention has a passive connection mechanism that does not rely on the relative movement of an actuator inside a collar for connection to a catheter hub.
According to the present invention, there is provided a closed system catheter assembly according to claim 1.
The present invention further provides a method of making a safety catheter assembly according to claim 12.

The instant invention is directed to a safety intravenous catheter assembly including a closed system catheter (CSC) assembly that has a catheter hub assembly and a needle insertion assembly. The catheter hub assembly has a catheter hub having a catheter tube extending from its distal end. The needle insertion assembly has a needle housing that includes a needle hub and a safety clip enclosed in a protective housing or clip guard removably housed within a compartment in a distal section of the needle housing. The safety clip in the clip guard fixedly holds the catheter hub when the CSC assembly is in a ready to use position or state, or simply a ready position. A needle, or needle cannula, has its proximal, or non-patient end, fixedly attached to the needle hub integrally formed inside the needle housing. The needle extends from the needle hub such that its patient end passes through the catheter with its bevel sharp distal tip extending beyond the distal end of the catheter. The proximal end of the needle may be flush with or extends slightly beyond the proximal end of the needle hub into a flashback chamber in the proximal section of the needle housing. A flashback plug is fitted to the open proximal end of the flashback chamber so that air in the cannula of the needle and flashback chamber may pass through while fluid that flows into the flashback chamber is contained therein.

The safety clip has two arms that extend from a base having an opening through which the needle passes. The arms each have an elongated member that extends distally from the base to an elbow where the member is bent substantially orthogonal to the elongated member to form a mid-section having an end curved to form a distal gripper. The gripper may be a hook like finger or a jaw. The respective grippers at the distal ends of the elongated members are movable relative to each other and are adapted to grasp the proximal end of the catheter hub in the ready to use position. At least one flange or protuberance may be formed at the open proximal end of the catheter hub to facilitate the grasping of the catheter hub by the grippers. The elongated members are naturally inwardly biasing such that in their natural state, the members pivot toward each other. The arms further have respective barriers proximal their corresponding elbows that naturally bias inwardly toward the longitudinal axis of the safety clip.

The barriers are biased outwardly away from each other when a needle, or more precisely the shaft of the needle, passes therebetween such that the distal grippers of the elongated members are forced inwardly to grasp the proximal end of the catheter hub. Thus, so long as the distal tip of the needle has not been retracted to a position proximal the naturally inwardly biasing barriers of the safety clip, the catheter hub is fixedly held to the safety clip. The sharp distal tip of the needle thus is prevented from being exposed to the environment.

To further ensure that the needle tip is continuously shielded from the environment, the safety clip is housed and fixedly held in the clip guard. The clip guard has a proximal portion that slidably form fits to an aperture at the distal end portion of the needle hub in the ready to use position, while the distal portion of the clip guard that shields the safety clip is housed in a similarly configured compartment in the distal section of the needle housing in the ready position.

The catheter hub of the CSC assembly has a distal end to which is attached the proximal end section of a catheter tube, or simply a catheter. An insert may be used to frictionally hold the proximal end section of the catheter to the catheter hub. In the ready position, the needle extends along the interior cavity of the catheter hub and slidably extends along the catheter with its sharp distal tip protrudes or extends past the distal end of the catheter. The catheter hub has a side opening to an extension integral of the catheter hub to which one end of an extension tubing is attached. The other end of the extension tubing has a connector mechanism adapted to be connected to a fluid store device. A pair of wings are integrally formed at the patient facing surface of the catheter hub to assist the clinician in positioning and holding the catheter hub assembly to the patient during use. The wings and catheter hub may be taped to the skin of the patient to hold the catheter hub assembly to the patient afterwards.

An interior cavity is formed inside the catheter hub between its distal end and open proximal end. An elastomeric septum is positioned proximal to the opening at the side wall that forms the side port extension to prevent fluid from flowing in the proximal direction towards the open proximal end of the catheter hub. When the needle is withdrawn from the catheter hub and the septum reseals, a fluid path is established between the vasculature of the patient to which the catheter is inserted and the extension tubing at the side opening of the catheter hub, and from there to the fluid store that may be connected to the extension tubing.

For an embodiment of the instant invention, a septum retainer may be inserted distally from the open proximal end of the catheter hub into its internal cavity so that the distal end of the septum retainer abuts against the proximal surface of the septum. A stop at the inner wall of the catheter hub prevents the septum from movement in the distal direction. Thus, the septum is held in place inside the catheter hub by the stop at the inner wall of the catheter hub and the septum retainer. An opening may be provided at the distal end of the septum retainer to enable the needle to pass into and through the septum.

In the ready position, the proximal end of the catheter hub is held to the clip guard by the distal grippers of the safety clip non-removably enclosed in the clip guard. When the needle is withdrawn from the patient and moves past the septum such that its distal tip is proximal the biasing barriers, no longer biased outwardly by the needle, those barriers would return to their natural inwardly biased state so that the distal grippers are released from the flange at the proximal end of the catheter hub. The clip guard may be removed from the catheter hub when the needle feature at the distal end of the needle comes into contact with the opening at the base of the safety clip, as the opening has a dimension preventing the needle feature from passing through. Further proximal movement of the needle relative to the catheter hub separates the safety clip and the clip guard non-removably attached thereto from the catheter hub assembly. As the needle is removed from the catheter hub, the needle housing is moved in the proximal direction relative to the catheter hub to pull the needle away from the catheter hub. Since the safety clip is non-removably enclosed in the clip guard, the contaminated distal tip of the needle continues to be shielded from the environment by the clip guard as the needle is withdrawn from the catheter hub into the safety clip. The distal tip of the needle is thus continuously shielded from when the CSC assembly is in the ready position to when it is in the safe position, whereby the distal tip of the needle is entrapped in the safety clip.

### Brief Description of the Figures

The present invention will become apparent and the invention itself will be best understood with reference to the following description of the invention taken in conjunction with the accompanying drawings, wherein:
Fig. 1 is an overall view of a closed system catheter (CSC) assembly of the instant invention;
Fig. 2 is the CSC assembly of Fig. 1 but with the needle sheath protecting the catheter and needle before use removed;
Fig. 3 is a top cross-sectional view of the inventive CSC assembly;
Fig. 4 is an enlarged view of the catheter hub and the distal portions of the clip guard and the safety clip of the needle insertion assembly;
Fig. 5 is a perspective view of an exemplar safety clip of the instant invention relative to the proximal end of the catheter hub;
Fig. 6 is a cut-away view of the proximal portion of the exemplar safety clip non-removably anchored to the clip guard;
Fig. 7 is a perspective view of the catheter hub assembly of the CSC assembly of the instant invention;
Fig. 8 is an exploded view of the different components of the Fig. 7 catheter hub assembly;
Fig. 9 is a cross-sectional side view of the catheter hub shown in Figs. 7-8;
Fig. 10 shows the CSC assembly in a ready to use position;
Fig. 11 shows the CSC assembly with the needle being removed from the catheter;
Fig. 12 shows the needle having been substantially separated from the catheter hub but the clip guard remains positioned relative to the catheter hub; and
Fig. 13 shows the needle insertion assembly separated from the catheter hub assembly.

### Detailed Description of the Invention

The instant application relates to provisional application US 63/227,508 filed July 30, 2021 and assigned to the assignee of the instant application.

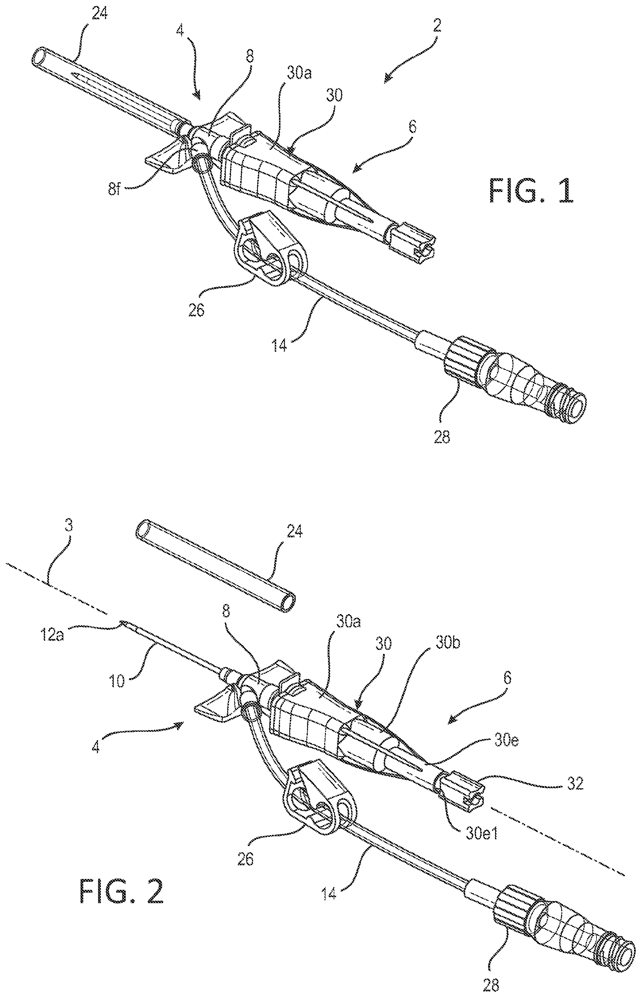

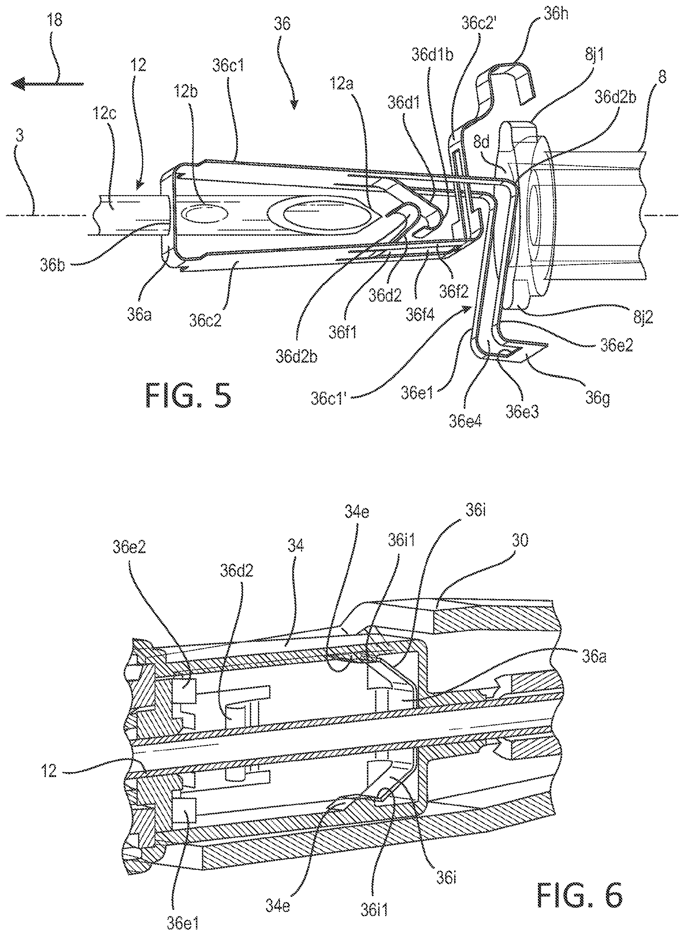

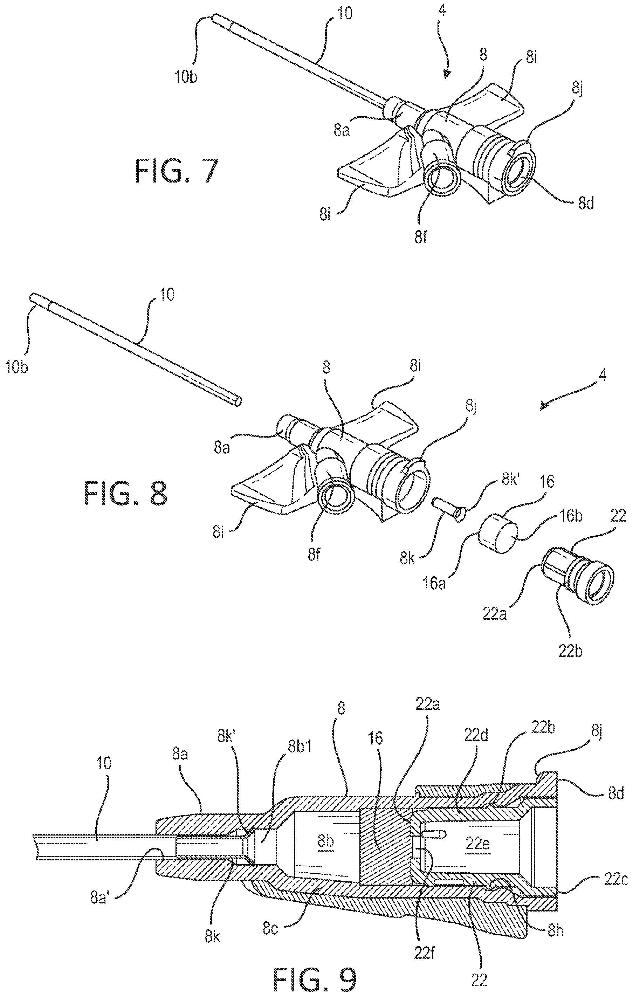

As shown in Figs. 1-4, the closed system catheter (CSC) assembly 2 is comprised of a catheter hub assembly 4 and a needle insertion assembly 6 lying coaxially along a longitudinal axis 3. Catheter hub assembly 4 has a catheter hub 8 that has a distal section 8a where a catheter tube, or simply catheter 10, is attached via its proximal end 10a. An insert (Fig. 8) may be used to frictionally attach proximal end 10a of catheter 10 to distal section 8a of catheter hub 8. Alternatively, the proximal end of the catheter may be attached to the distal end of the catheter hub by bonding as is well known. A needle 12 extends through catheter 10 with its bevel sharp distal tip 12a extending beyond a distal end 10b of catheter 10. As best shown in Fig. 5, a needle feature 12b that has a dimension or configuration different from the shaft 12c of needle 12 is provided proximate to the sharp distal tip 12a of needle 12.

Catheter hub 8 has an interior cavity 8b defined by a wall 8c extending from distal section 8a to an open proximal end 8d. A side opening 8e at wall 8c connects the interior cavity 8b to a side port extension 8f at catheter hub 8. For discussion purpose, side port extension 8f may be referred to simply as a side port or an extension of the catheter hub. One end of an extension tubing 14 is fixedly fitted to the open end of extension 8f. The other end of the extension tubing 14 has fitted thereto a connector 28 adapted to connect to a fluid store device such as a syringe, a fluid line, a pump or alike as is well known, so that fluid may be input to the internal cavity 8b of catheter hub 8 from the fluid store. A clamp 26 adapted to squeeze shut tubing 14 to prevent fluid flow therealong is slidably fitted along tubing 14. Catheter hub 8 is shown in greater detail in Figs. 7-9.

A septum 16 made of an elastomeric material as is well known is positioned in internal cavity 8b proximal to side opening 8e to prevent fluid from flowing in the proximal direction as indicated by directional arrow 18. Elastomeric septum 16 has the quality of allowing a needle such as needle 12 to pierce through it and, after the needle is removed, reseals itself. A shoulder 8g formed circumferentially at the inner surface of wall 8c provides a stop to the distal end 16a of septum 16 from moving in the distal direction as indicated by directional arrow 20.

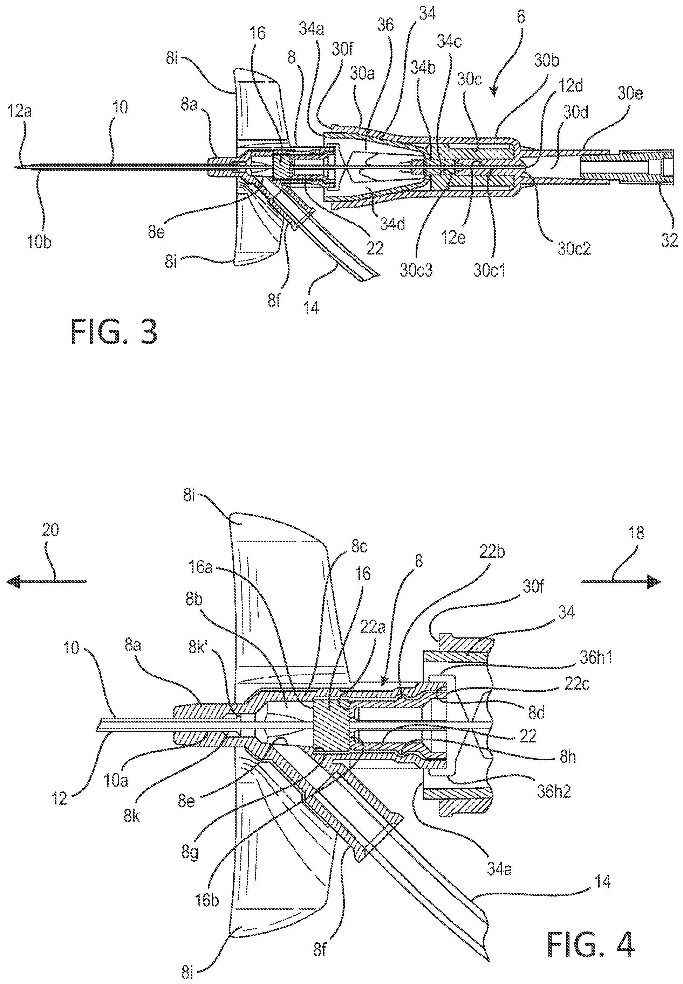

With reference to Figs. 3-4 and 7-9, a septum retainer 22 is shown inserted into open proximal end 8d of catheter hub 8 such that its distal end 22a, or the surface at the distal end, abuts against the proximal end 16b of septum 16 to hold septum 16 in place. Septum retainer 22 has a cup-shaped configuration where its distal end corresponds to the bottom of the cup and the circumferential wall 22d extending therefrom defines an interior cavity 22e for the septum retainer. As best shown in Fig. 9, an opening 22f is provided at distal end 22a with a dimension that allows needle 12 to pass through. Septum retainer 22 may be fixedly held in the proximal section of catheter hub 8 by a circumferential protuberance 22b at the septum retainer mating with an infernal circumferential groove 8h formed at the inner wall of catheter hub 8. For the embodiment shown, septum retainer 22 has a proximal end 22c that is flush with the proximal end 8d of catheter hub 8. However, it should be appreciated that the proximal end of septum retainer 22 may extend proximally beyond or distally inwardly from the proximal end 8d of catheter hub 8. At least one flange may be provided at proximal end 8d of catheter hub 8 to receive distal grippers of the safety clip as will be discussed, *infra.*

Extending orthogonally at the underside of catheter hub 8 are two wings 8i that may be employed to hold the catheter hub against the skin of the patient when using the CSC assembly 2. As shown in Figs. 1 and 2, prior to use, catheter 10 and needle 12 slidably extending therethrough are shielded by a needle sheath 24, shown to be removed in Fig. 2. Figs. 1-2 show CSC assembly 2 in the ready to use position or state, or simply the ready position. In Figs. 7 and 8, open proximal end 8d of catheter hub 8 is shown to have a flange 8j that may be used to couple to the distal grippers of the safety clip to be discussed below. Although one flange is shown, it should be appreciated that the flange may be one continuous circumferential flange, or multiple partial circumferential flanges such as flanges 8j1 and 8j2 shown in Fig. 5 that may be grasped by the grippers of the safety clip.

Figs. 8 and 9 show an insert 8k pressure fitted into the passage 8a' defined by wall 8c that extends through distal section 8a in catheter hub 8. Insert 8k is inserted into passage 8a' to frictionally press hold the proximal end of catheter 10 to the distal end of catheter hub 8. When fully fitted to the passage, the tapered proximal end 8k' of insert 8k makes contact with the smaller distal section 8b1 of internal cavity 8b of catheter hub 8 to prevent fluid from passing.

With reference to the Figs. 1-3 embodiment shown, needle insertion assembly 6 includes a needle housing 30 having a distal section 30a configured to have four walls each taper outwardly in the distal direction. Needle housing 30 has a mid-section 30b that includes an integral internal needle hub 30c. Needle hub 30c has an internal passageway 30c1 into which a proximal end 12e of needle 12 is fixedly attached, by bonding or frictional fit as is well known. Proximal end 12d of needle 12 may be flush with the proximal end 30c2 of needle hub 30c. Proximal end 12d of needle 12 feeds into an internal or flashback chamber 30d at the proximal section 30e of needle housing 30. The open proximal end 30e1 of proximal section 30e is fitted with a flash plug 32 that may be made of an air permeable hydrophilic material to allow air to pass but inhibits the passage of fluid as is well known. Air that resides in flashback chamber 30d is pushed through flash plug 32 by the incoming blood until the blood reaches the hydrophilic material of the flash plug which prevents the blood from passing.

Removably housed in the compartment defined by distal section 30a of needle housing 30 is a needle tip protector guard, or clip guard 34. Clip guard 34 is configured to be form fittingly housed within the compartment defined by distal section 30a. As shown in Fig. 3, clip guard 34 has an open distal end 34a that tapers inwardly in the proximal direction to a proximal back end 34b having a proximal extension 34c that matingly fits into an aperture 30c3 at the distal end portion of needle hub 33 in the ready position. Distal end 34a of clip guard 34 extends distally from the distal end 30f of needle housing 30 as per shown in Figs. 3 and 4 exemplar embodiment. In practice, it should be appreciated that distal end 34a of clip guard 34 may be flush with the distal end 30f of needle housing 30 or proximal to the distal end of the needle housing in the ready position. The thing to note is that distal end 34a of clip guard 34 encloses the distal grippers of the safety clip housed in clip guard 34, as discussed herein.

As shown in Fig. 3, fixedly enclosed within its interior chamber 34d in clip guard 34 is a needle protection device or safety clip 36. One embodiment of safety clip 36 is shown in detail in Fig. 5. With reference to Figs. 3 and 4, CSC catheter assembly 2 is shown to have the proximal end 8d of catheter hub 8 covered by the open distal end 34a of clip guard 34. The remaining portion of clip guard 34 is received in the compartment at the distal section 30a of needle housing 30.

Fig. 5 shows the safety clip 36 relative to the proximal section of catheter hub 8. Needle safety clip 36 is shown to have two arms 36c1 and 36c2 that extend substantially transversely in the distal direction from opposite ends of a base 36a. An opening 36b formed at base 36a has a dimension that allows needle 12 to pass but prevents needle feature 12b from passing in the proximal direction as indicated by directional arrow 18. Respective mid-sections 36d1 and 36d2 of the arms 36c1 and 36c2 are bent toward the center of safety clip 36, with their corresponding bent ends 36d1b and 36d2b curved to form respective naturally inwardly biasing members, or barriers 36d1b and 36d2b.

Further with reference to Fig. 5, with the mid-sections 36d1 and 36d2 bent inwardly from arms 36c1 and 36c2, slots along arms 36c1 and 36c2 are formed by the parallel rails left with the inwardly bending of the mid-sections. In particular, parallel rails 36e1 and 36e2 extend from the mid-section of arm 36c1 to a cross end 36e3 that connects the rails to define a slot 36e4 with a width that allows needle 12 to pass through. As shown, rails 36e1 and 36e2 coplanarly extend distally to a distance proximal the proximal end 8d of catheter hub 8, and are then bent slightly off at right angle downwardly to form a downward section 36c1'. Similarly, arm 36c2 is bent in a reverse manner such that rails 36f1 and 36f2 are bent upwardly at a slightly off right angle from the longitudinal portion of arm 36c2 to form an upright section 36c2' that defines a slot 36f4 with space sufficient to allow needle 12 to pass through. Section 36c2' is formed at a distance proximal from section 36c1'. Slot 36f4 of upright section 36c2' has a width that is smaller than the width of slot 36e4 of section 36c1' so that upright section 36c2' is freely movable within slot 36e4. The end portion of rails 36e1 and 36e2 are further bent in the distal direction substantially transverse to section 36c1' to form a lower jaw 36g adapted to press against flange 8j2 of catheter hub 8, while the distal end of arm 36c2 is configured as a hook like finger 36h adapted to grasp flange 8j1 to couple safety clip 36 to catheter hub 8.

For the embodiment shown in Figs. 3 and 4, the lower jaw of arm 36c1 is replaced with a hook like finger such as 36h. Thus, the safety clip 36 may have hook like fingers at the distal end of both of its arms as per shown in Figs. 3 and 4. Both of the hook fingers shown in Figs. 3-4 and the combination of hook finger and jaw shown in Fig. 5 may simply be referred to as the grippers of safety clip 36.

When in the safe position as shown in Fig. 5 where barriers 36d1b and 36d2b are in their natural inwardly biased positions since they are not biased by needle 12, the barriers effect a stop distal of the distal tip 12a of needle 12 to prevent needle 12 from re-emerging from safety clip 36 in the distal direction. At the same time, needle feature 12b prevents needle 12 from further proximal movement relative to safety clip 36 through opening 36b. Thus, the distal end portion including distal tip 12a of needle 12 is entrapped in safety clip 36. As shown, barrier 36d1b is distal to barrier 36d2b, with distal tip 12a of needle 12 proximal to both barriers 36d1b and 36d2b. Fig. 5 shows the CSC assembly 2 in the safe state or position, as the potentially contaminated distal tip 12a of the needle 12 is trapped inside safety clip 36.

With reference to Fig. 3 and the detailed showing of safety clip 36 in Fig. 5, when CSC assembly 2 is in the ready position, barriers 36d1b and 36d2b are biased by needle 12 away from the longitudinal axis 3 of the CSC assembly. Needle 12 also passes slots 36f4 and 36e4 of arms 36c2 and 36c2, respectively, into catheter hub 8. With barriers 36d1 and 36d2 biased outwardly, transverse portions 36c1' and 36c2' would move in opposite directions away from the longitudinal axis 3 such that hook finger 36h would grasp flange 8j1 and jaw 36g would press against flange 8j2 of catheter hub 8, such that safety clip 36 and catheter hub 8 are coupled to each other. Although a combination of hook and jaw is shown in the embodiment of Fig. 5, it should be appreciated that In practice, the arms each may have a hook finger at their distal end as per shown in Figs. 3-4, or some other finger gripping mechanisms that allow the safety clip to grippingly hold catheter hub 8. Thus, the proximal end of the catheter hub is fixedly held by the distal grippers of the safety clip in the ready position. It should further be appreciated that safety clip 36 as described above may be adapted to be used for safety catheter assembles other than CSC catheter assemblies.

Figs. 3 and 4 show safety clip 36 and catheter hub 8 coupled to each other by means of dual finger hook grippers 36 h1 and 36h2 instead of the combination hook and jaw as discussed above with reference to Fig. 5. Functionally, the finger hook grippers in Figs. 3 and 4 operate in the same manner as the grippers discussed with reference to Fig. 5, except that the opposite flanges 8j1 and 8j2 of the catheter hub 8 each are grasped by a corresponding one of grippers 36h1 and 36h2.

Fig. 3 shows safety clip 36 being housed or enclosed in clip guard 34. Clip guard 34 in turn is received in the compartment defined by distal section 30a of needle housing 30. Safety clip 36 has the same parts as the safety clip described in Fig. 5, except that the distal grippers are both hook type gripper mechanisms. To maintain safety clip 36 within chamber 34d of clip guard 34, a pair of opposing projections or tangs 36i extending from base 36a as per shown in Fig. 6 are added to safety clip 36. Undercuts 34e or other structure adapted to coact with tangs 36i are formed at the opposing inner walls of clip guard 34. When safety clip 36 is inserted into chamber 34b, tangs 36i, when coming into contact with the tapered distal surfaces of undercuts 34e, would bend inwardly to pass the undercuts. Once past the undercuts, tangs 36i revert back to their unbiased natural state so that their respective edges 36i1 would abut against the proximal back surfaces of the respective undercuts 34e to prevent safety clip 36 from moving in the distal direction relative to clip guard 34. As a result, safety clip 36 is non-removably enclosed in chamber 34b of clip guard 34. Note that the opposing tangs 36i extend from opposing sides of base 36a of safety clip 36 while the arms 36c1 and 36c2 of safety clip 36 extend from the other opposing sides of base 36a.

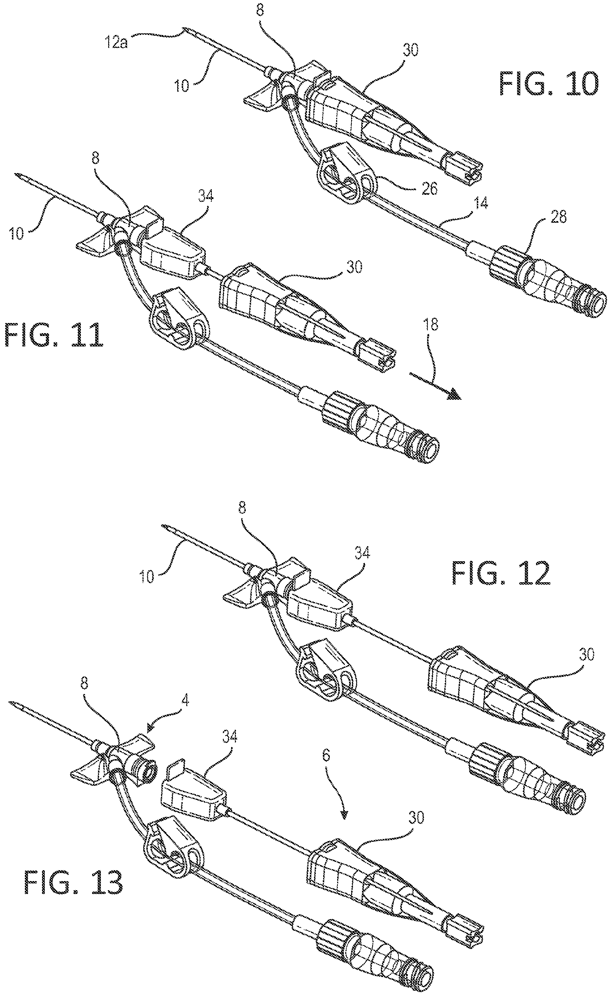

The operation of the safety catheter assembly of the instant invention is illustrated in Figs. 10-13. In the ready position as shown in Fig. 10, catheter hub 8 is coupled to clip guard 34 by the distal grippers of safety clip 36 fixedly holding the proximal end 8d of catheter hub 8. After the needle is inserted into the appropriate vein or artery of the patient followed by the insertion of catheter 10, the clinician would withdraw needle12 by moving needle housing 30 in the proximal direction as indicated by directional arrow 18 relative to catheter hub 8, as per shown in Fig. 11. With further movement of needle housing 30 in the proximal direction relative to catheter hub 8, the needle feature 12b (Fig. 5) comes into contact with opening 36b at base 36a of safety clip 36 and the distal tip 12a of needle 12 is positioned proximal of barriers 36b1 and 36b2. No longer biased by needle 12, barriers 36b1 and 36b2 return to their natural inwardly biased state such that the distal grippers are released from proximal end 8d of catheter hub 8. Since safety clip 36 is non-removably held to clip guard 34, upon further proximal movement of needle housing 30 relative to catheter hub 8, clip guard 34 is removed from the catheter hub, with safety clip 34 enclosed therein and distal tip 12a of needle 12 entrapped in the safety clip. Fig. 13 shows needle housing assembly 6 and catheter hub assembly 4 separated from each other.

The CSC assembly 2 shown in Fig. 13 may be considered to be in a safe position or state in that the distal tip 12a of needle 12 is trapped in safety clip 36 enclosed by clip guard 34 within needle housing 30 of the needle insertion assembly 6. Thus, throughout the life of CSC assembly 2, from shipping to use to disposal, or from the ready position to the safe position, the distal portion of the needle and more particularly the sharp distal end of the needle is prevented from being exposed to the environment.

## Claims

1. A closed system catheter assembly including a catheter hub (8) having a body including a distal end (8a) having extending distally therefrom a catheter (10) having a distal tip (10b), an open proximal end (8d) and a wall (8c) extending between the distal end (8a) and the proximal end (8d) defining an internal cavity (8b), a side port (8f) extending from an opening (8e) at the wall (8c) between the distal end (8a) and the proximal end (8d), wherein the closed system catheter assembly comprises:
a needle insertion assembly (6) including a needle housing (30) having a needle hub (30c) separating a distal section (30a) and a proximal section (30e) of the needle housing (10), the needle hub (30c) having secured thereto a proximal end (12e) of a needle (12) having a sharp distal tip (12a), the needle (12) establishing a fluid passageway between the sharp distal tip (12a) and the proximal section (30e) of the needle housing (30);
a clip guard (34); and
a safety clip (36) non-removably housed in the clip guard (34) slidably mounted along the needle (12), the safety clip (36) including a base (36b) having two arms (36c1, 36c2) extending distally from the base (36b), the two arms (36c1, 36c2) having respective distal grippers (36h, 36g) and respective naturally inwardly biasing members (36d1b, 36d2b);
wherein the needle (12) extends through the catheter hub (8) and its distal sharp tip (12a) extends past the distal end (10b) of the catheter (10), the proximal end (8d) of the catheter hub (8) held by the distal grippers (36h, 36g) of the two arms (36c1, 36c2) of the safety clip (36) and the clip guard (34) enclosed by the distal section (30a) of the needle housing (30) when the catheter assembly is in a ready position.

2. The closed system catheter assembly of claim 1, further comprising:
an elastomeric septum (16) fitted inside the catheter hub (8) proximal to the opening (8e) of the side port (8f), the septum (16) adapted to be pierced by the needle (12) in the ready position and reseals when the needle is removed from the septum (16).

3. The closed system catheter assembly of claim 1, further comprising:
a septum retainer (22) non-removably inserted into the open proximal end (8d) of the catheter hub (8), the septum retainer (22) having a distal end (22a) in abutment with a proximal end (16b) of a septum (16) fitted inside the catheter hub (8) to prevent proximal movement of the septum (16).

4. The closed system catheter assembly of claim 3, wherein the septum (16) has a distal end (16a) in non-movable contact with an inner wall stop (8g) proximal to the opening (8e) of side port (8f) to hold the septum (16) in place inside the catheter hub (8) such that after the needle (12) is withdrawn from the catheter (10), a fluid communication path is established between the catheter (10) and the side port (8f) through the internal cavity (8b) of the catheter hub (8).

5. The closed system catheter assembly of claim 3, wherein the distal end (22a) of the septum (16) retainer has an opening (22f) through which the needle (12) passes to pierce the septum (16) when the catheter assembly is in the ready position.

6. The closed system catheter assembly of claim 1, wherein the clip guard (34) comprises a proximal section (30d) configured to fittingly mate to a distal aperture (30c3) of the needle hub (30c) and a chamber (34d) for enclosing the safety clip (36), the clip guard (34) removably form fits into the distal section (30a) of the needle housing (30) in the ready position.

7. The closed system catheter assembly of claim 1, wherein the two arms (36c1, 36c2) are one and other elongated members (36c1,36c2) each extend longitudinally away from the base (36b) to corresponding elbows where the elongated members (36c1,36c2) are bent substantially orthogonally toward each other to form respective bent portions having the distal grippers (36h, 36g), one of the one and other elongated members (36c1,36c2) having a slot (36e4) through which the bent portion of the other of the one and other elongated members (36c1,36c2) passes, the respective naturally inwardly biasing members (36d1b, 36d2b) are provided at the one and other elongated members (36c1,36c2) proximal to their corresponding elbows so that, when the catheter assembly is in the ready position, the respective naturally inwardly biasing members (36d1b, 36d2b) are biased by the needle passing therebetween to bias the elongated members (36c1,36c2) outwardly to cause the respective bent portions to move toward each other such that the proximal end (8d) of the catheter hub (8) is grasped by the distal grippers (36h, 36g).

8. The closed catheter assembly of claim 1, wherein the clip guard (34) is configured to form fittingly housed in the distal section (30a) of the needle housing (10), the clip guard (34) having an open distal end (34a); and
wherein the safety clip (36) is non-removably enclosed in a chamber of the clip guard (34) such that the distal grippers (36h, 36g) of the safety clip (36) are enclosed by the distal end of the clip guard (34).

9. The closed system catheter assembly of claim 1, wherein in a safe position, the distal tip (12a) of the needle (12) is withdrawn proximally past the biasing members (36d1b, 36d2b) so that the biasing members (36d1b, 36d2b) would return to their natural inward position to provide a stop to prevent the distal tip of the needle from reemerging out of the safety clip (36).

10. The closed system catheter assembly of claim 1, wherein the base (36b) of the safety clip (36) is fixedly held to a proximal portion of the clip guard (34) such that the safety clip (36) is non-removably held inside the clip guard (34), the clip guard (34) movable relative to the needle housing (10).

11. The closed system catheter assembly of claim 1, wherein the proximal section (30e) of needle housing (10) is a flash chamber with a flash plug at a proximal opening of the needle housing (10) that allows air to pass but prevents fluid from passing out of the proximal section; and
wherein the catheter hub (8) has side wings (8i) integrally extending from the outside wall of the catheter hub (8) and an extension tubing (14) connected to the side port.

12. A method of making a safety catheter assembly, comprising the steps of:
providing a catheter hub (8) having a body including a distal end (8a), an open proximal end (8d) and a wall (8c) extending between the distal end (8a) and the proximal end (8d) defining an internal cavity (8b);
extending a catheter (10) having a distal tip (10a) from the distal end (8a) of the catheter hub (8);
providing a needle housing (10) having a distal section (30a), a proximal section (30e) and a needle hub (30c) sandwiched between the distal section (30a) and the proximal section (30e),
attaching a needle (12) having a sharp distal tip (12a) and a proximal end (12e) to the needle hub (30c) to establish a fluid passageway between the sharp distal tip (12a) and the proximal section (30e) of the needle housing (10);
removably positioning a clip guard (34) in the distal section (30a) of the needle housing (10);
slidably mounting a safety clip (36) along the needle (12);
non-removably housing the safety clip (36) in the clip guard (34), the safety clip (36) including a base (36b) having two arms (36c1, 36c2) extending distally from the base (36b), the two arms (36c1, 36c2) having respective distal grippers (36h, 36g) and respective naturally inwardly biasing members (36d1b, 36d2b); and
extending the needle (12) through the catheter hub (8) and its distal sharp tip (12a) past the distal end (10b) of the catheter (10) such that the proximal end (8d) of the catheter hub (8) is fixedly held by the distal grippers (36h, 36g) of the two arms (36c1, 36c2) of the safety clip (36) and the clip guard (34) is enclosed in the distal section (30a) of the needle housing (30) when the catheter assembly is in a ready position.

13. The method of making a safety catheter assembly of claim 12, further comprising the steps of:
extending a side port (8f) from an opening (8e) at the wall between the distal end (8a) and the proximal end (8d) at the catheter hub (8);
fitting an elastomeric septum (16) inside the catheter hub (8) proximal to the opening (8e) of the side port (8f), the septum (16) adapted to be pierced by the needle (12) in the ready position and reseals when the needle (12) is removed from the septum (16).

14. The method of making a safety catheter assembly of claim 12, further comprising the steps of:
non-removably inserting a septum retainer (22) into the open proximal end (8d) of the catheter hub (8);
abutting a distal end (22a) of the septum retainer (22) with a proximal end (16b) of a septum (16) fitted inside the catheter hub (8) to prevent proximal movement of the septum (16);
providing an inner wall stop (8g) proximal to the opening (8e) of side port (8f) to prevent distal movement of the septum (16) relative to the catheter hub (8) hub so that the septum (16) is held in place inside the catheter hub (8).

15. The method of making a safety catheter assembly of claim 12, further comprising the step of:
configuring the clip guard (34) to have a proximal extension (34c) fittingly matable to a distal aperture (30c3) of the needle hub (30c) and a chamber (34d) for enclosing the safety clip (36), the clip guard (34) form fitting into the distal section (30a) of the needle housing (10) in the ready position.

## Patentansprüche

1. Katheteranordnung mit geschlossenem System, die eine Katheternabe (8) beinhaltet, die einen Körper aufweist, der ein distales Ende (8a) mit einem sich davon distal erstreckenden Katheter (10) mit einer distalen Spitze (10b), ein offenes proximales Ende (8d) und eine Wand (8c) beinhaltet, die sich zwischen dem distalen Ende (8a) und dem proximalen Ende (8d) erstreckt und einen inneren Hohlraum (8b) definiert, wobei sich ein Seitenanschluss (8f) von einer Öffnung (8e) an der Wand (8c) zwischen dem distalen Ende (8a) und dem proximalen Ende (8d) erstreckt, wobei die Katheteranordnung mit geschlossenem System Folgendes umfasst:
eine Nadeleinführungsanordnung (6), die ein Nadelgehäuse (30) beinhaltet, das eine Nadelnabe (30c) aufweist, die eine distale Sektion (30a) und eine proximale Sektion (30e) des Nadelgehäuses (10) trennt, wobei die Nadelnabe (30c) ein daran befestigtes proximales Ende (12e) einer Nadel (12) mit einer scharfen distalen Spitze (12a) aufweist, wobei die Nadel (12) einen Fluiddurchgang zwischen der scharfen distalen Spitze (12a) und der proximalen Sektion (30e) des Nadelgehäuses (30) herstellt;
einen Clipschutz (34); und
einen Sicherheitsclip (36), der nicht entfernbar in dem Clipschutz (34) untergebracht ist, der verschiebbar entlang der Nadel (12) montiert ist, wobei der Sicherheitsclip (36) eine Basis (36b) beinhaltet, die zwei Arme (36c1, 36c2) aufweist, die sich distal von der Basis (36b) erstrecken, wobei die zwei Arme (36c1, 36c2) jeweilige distale Greifer (36h, 36g) und jeweilige natürlich einwärts vorspannende Elemente (36d1b, 36d2b) aufweisen;
wobei sich die Nadel (12) durch die Katheternabe (8) erstreckt und sich ihre distale scharfe Spitze (12a) über das distale Ende (10b) des Katheters (10) hinaus erstreckt, wobei das proximale Ende (8d) der Katheternabe (8) von den distalen Greifern (36h, 36g) der zwei Arme (36c1, 36c2) des Sicherheitsclips (36) gehalten wird und der Clipschutz (34) von der distalen Sektion (30a) des Nadelgehäuses (30) umschlossen ist, wenn sich die Katheteranordnung in einer Bereitschaftsposition befindet.

2. Katheteranordnung mit geschlossenem System nach Anspruch 1, ferner umfassend:
ein elastomeres Septum (16), das in dem Inneren der Katheternabe (8) proximal zu der Öffnung (8e) des Seitenanschlusses (8f) eingepasst ist, wobei das Septum (16) dazu angepasst ist, von der Nadel (12) in der Bereitschaftsposition durchstochen zu werden, und sich wieder abdichtet, wenn die Nadel von dem Septum (16) entfernt wird.

3. Katheteranordnung mit geschlossenem System nach Anspruch 1, ferner umfassend:
einen Septumhalter (22), der nicht entfernbar in das offene proximale Ende (8d) der Katheternabe (8) eingeführt ist, wobei der Septumhalter (22) ein distales Ende (22a) aufweist, das an ein proximales Ende (16b) eines Septums (16) anstößt, das in dem Inneren der Katheternabe (8) eingepasst ist, um eine proximale Bewegung des Septums (16) zu verhindern.

4. Katheteranordnung mit geschlossenem System nach Anspruch 3, wobei das Septum (16) ein distales Ende (16a) in unbeweglichem Kontakt mit einem Innenwandanschlag (8g) proximal zu der Öffnung (8e) des Seitenanschlusses (8f) aufweist, um das Septum (16) in dem Inneren der Katheternabe (8) an Ort und Stelle zu halten, sodass, nachdem die Nadel (12) aus dem Katheter (10) herausgezogen wird, ein Fluidkommunikationsweg zwischen dem Katheter (10) und dem Seitenanschluss (8f) durch den inneren Hohlraum (8b) der Katheternabe (8) hergestellt wird.

5. Katheteranordnung mit geschlossenem System nach Anspruch 3, wobei das distale Ende (22a) des Halters des Septums (16) eine Öffnung (22f) aufweist, durch die die Nadel (12) verläuft, um das Septum (16) zu durchstechen, wenn sich die Katheteranordnung in der Bereitschaftsposition befindet.

6. Katheteranordnung mit geschlossenem System nach Anspruch 1, wobei der Clipschutz (34) eine proximale Sektion (30d), die dazu konfiguriert ist, passend zu einem distalen Loch (30c3) der Nadelnabe (30c) zu passen, und eine Kammer (34d) zum Umschließen des Sicherheitsclips (36) umfasst, wobei der Clipschutz (34) in der Bereitschaftsposition entfernbar in die distale Sektion (30a) des Nadelgehäuses (30) formschlüssig passt.

7. Katheteranordnung mit geschlossenem System nach Anspruch 1, wobei die zwei Arme (36c1, 36c2) ein und ein anderes längliches Element (36c1,36c2) sind, die sich jeweils in Längsrichtung von der Basis (36b) weg zu entsprechenden Winkelstücken erstrecken, wo die länglichen Elemente (36c1,36c2) im Wesentlichen orthogonal zueinander gebogen sind, um jeweilige gebogene Abschnitte mit den distalen Greifern (36h, 36g) auszubilden, wobei eines des einen und des anderen länglichen Elements (36c1,36c2) einen Schlitz (36e4) aufweist, durch den der gebogene Abschnitt des anderen des einen und des anderen länglichen Elements (36c1,36c2) verläuft, wobei die jeweiligen natürlich einwärts vorspannenden Elemente (36d1b, 36d2b) an dem einen und dem anderen länglichen Element (36c1,36c2) proximal zu ihren entsprechenden Winkelstücken bereitgestellt sind, sodass, wenn sich die Katheteranordnung in der Bereitschaftsposition befindet, die jeweiligen natürlich einwärts vorspannenden Elemente (36d1b, 36d2b) durch die dazwischen verlaufende Nadel vorgespannt werden, um die länglichen Elemente (36c1,36c2) nach außen vorzuspannen, um zu bewirken, dass sich die jeweiligen gebogenen Abschnitte aufeinander zubewegen, sodass das proximale Ende (8d) der Katheternabe (8) von den distalen Greifern (36h, 36g) ergriffen wird.

8. Geschlossene Katheteranordnung nach Anspruch 1, wobei der Clipschutz (34) dazu konfiguriert ist, sich formschlüssig in der distalen Sektion (30a) des Nadelgehäuses (10) untergebracht auszubilden, wobei der Clipschutz (34) ein offenes distales Ende (34a) aufweist; und
wobei der Sicherheitsclip (36) nicht entfernbar in einer Kammer des Clipschutzes (34) umschlossen ist, sodass die distalen Greifer (36h, 36g) des Sicherheitsclips (36) von dem distalen Ende des Clipschutzes (34) umschlossen sind.

9. Katheteranordnung mit geschlossenem System nach Anspruch 1, wobei in einer sicheren Position die distale Spitze (12a) der Nadel (12) proximal über die vorspannenden Elemente (36d1b, 36d2b) hinaus zurückgezogen wird, sodass die vorspannenden Elemente (36d1b, 36d2b) in ihre natürliche einwärts gerichtete Position zurückkehren würden, um einen Anschlag bereitzustellen, um zu verhindern, dass die distale Spitze der Nadel wieder aus dem Sicherheitsclip (36) austritt.

10. Katheteranordnung mit geschlossenem System nach Anspruch 1, wobei die Basis (36b) des Sicherheitsclips (36) fest an einem proximalen Abschnitt des Clipschutzes (34) gehalten wird, sodass der Sicherheitsclip (36) nicht entfernbar in dem Inneren des Clipschutzes (34) gehalten wird, wobei der Clipschutz (34) relativ zu dem Nadelgehäuse (10) beweglich ist.

11. Katheteranordnung mit geschlossenem System nach Anspruch 1, wobei die proximale Sektion (30e) des Nadelgehäuses (10) eine Blutrückflusskammer mit einem Blutfängerstopfen an einer proximalen Öffnung des Nadelgehäuses (10) ist, der Luft passieren lässt, aber verhindert, dass Fluid aus dem proximalen Abschnitt austritt; und
wobei die Katheternabe (8) Seitenflügel (8i), die sich einstückig von der Außenwand der Katheternabe (8) erstrecken, und einen Verlängerungsschlauch (14), der mit dem Seitenanschluss verbunden ist, aufweist.

12. Verfahren zum Herstellen einer Sicherheitskatheteranordnung, umfassend die folgenden Schritte:
Bereitstellen einer Katheternabe (8), die einen Körper aufweist, der ein distales Ende (8a), ein offenes proximales Ende (8d) und eine Wand (8c) beinhaltet, die sich zwischen dem distalen Ende (8a) und dem proximalen Ende (8d) erstreckt und einen inneren Hohlraum (8b) definiert;
Verlängern eines Katheters (10), der eine distale Spitze (10a) aufweist, von dem distalen Ende (8a) der Katheternabe (8);
Bereitstellen eines Nadelgehäuses (10), das eine distale Sektion (30a), eine proximale Sektion (30e) und eine Nadelnabe (30c) aufweist, die zwischen der distalen Sektion (30a) und der proximalen Sektion (30e) sandwichartig angeordnet ist,
Anbringen einer Nadel (12), die eine scharfe distale Spitze (12a) und ein proximales Ende (12e) aufweist, an der Nadelnabe (30c), um einen Fluiddurchgang zwischen der scharfen distalen Spitze (12a) und der proximalen Sektion (30e) des Nadelgehäuses (10) herzustellen;
entfernbares Positionieren eines Clipschutzes (34) in der distalen Sektion (30a) des Nadelgehäuses (10);
verschiebbares Montieren eines Sicherheitsclips (36) entlang der Nadel (12);
nicht entfernbares Unterbringen des Sicherheitsclips (36) in dem Clipschutz (34), wobei der Sicherheitsclip (36) eine Basis (36b) beinhaltet, die zwei Arme (36c1, 36c2) aufweist, die sich distal von der Basis (36b) erstrecken, wobei die zwei Arme (36c1, 36c2) jeweilige distale Greifer (36h, 36g) und jeweilige natürlich einwärts vorspannende Elemente (36d1b, 36d2b) aufweisen; und
Verlängern der Nadel (12) durch die Katheternabe (8) und ihre distale scharfe Spitze (12a) über das distale Ende (10b) des Katheters (10) hinaus, sodass das proximale Ende (8d) der Katheternabe (8) von den distalen Greifern (36h, 36g) der zwei Arme (36c1, 36c2) des Sicherheitsclips (36) fest gehalten wird und der Clipschutz (34) in der distalen Sektion (30a) des Nadelgehäuses (30) umschlossen ist, wenn sich die Katheteranordnung in einer Bereitschaftsposition befindet.

13. Verfahren zum Herstellen einer Sicherheitskatheteranordnung nach Anspruch 12, ferner umfassend die folgenden Schritte:
Verlängern eines Seitenanschlusses (8f) von einer Öffnung (8e) an der Wand zwischen dem distalen Ende (8a) und dem proximalen Ende (8d) an der Katheternabe (8);
Einpassen eines elastomeren Septums (16) in dem Inneren der Katheternabe (8) proximal zu der Öffnung (8e) des Seitenanschlusses (8f), wobei das Septum (16) dazu angepasst ist, von der Nadel (12) in der Bereitschaftsposition durchstochen zu werden, und sich wieder abdichtet, wenn die Nadel (12) von dem Septum (16) entfernt wird.

14. Verfahren zum Herstellen einer Sicherheitskatheteranordnung nach Anspruch 12, ferner umfassend die folgenden Schritte:
nicht entfernbares Einführen eines Septumhalters (22) in das offene proximale Ende (8d) der Katheternabe (8);
Anstoßen eines distalen Endes (22a) des Septumhalters (22) an ein proximales Ende (16b) eines Septums (16), das in dem Inneren der Katheternabe (8) eingepasst ist, um eine proximale Bewegung des Septums (16) zu verhindern;
Bereitstellen eines Innenwandanschlags (8g) proximal zu der Öffnung (8e) des Seitenanschlusses (8f), um eine distale Bewegung des Septums (16) relativ zu der Katheternabe (8) zu verhindern, sodass das Septum (16) in dem Inneren der Katheternabe (8) an Ort und Stelle gehalten wird.

15. Verfahren zum Herstellen einer Sicherheitskatheteranordnung nach Anspruch 12, ferner umfassend die folgenden Schritte:
Konfigurieren des Clipschutzes (34), sodass er eine proximale Verlängerung (34c), die formschlüssig mit einem distalen Loch (30c3) der Nadelnabe (30c) zusammenpassen kann, und eine Kammer (34d) zum Umschließen des Sicherheitsclips (36) aufweist, wobei der Clipschutz (34) in der Bereitschaftsposition in die distalen Sektion (30a) des Nadelgehäuses (10) formschlüssig passt.

## Revendications

1. Ensemble cathéter à système fermé comprenant une embase de cathéter (8) possédant un corps comprenant une extrémité distale (8a) à partir de laquelle s'étend de manière distale un cathéter (10) possédant une pointe distale (10b), une extrémité proximale ouverte (8d) et une paroi (8c) s'étendant entre l'extrémité distale (8a) et l'extrémité proximale (8d) définissant une cavité interne (8b), un orifice latéral (8f) s'étendant à partir d'une ouverture (8e) au niveau de la paroi (8c) entre l'extrémité distale (8a) et l'extrémité proximale (8d), ledit ensemble cathéter à système fermé comprenant :
un ensemble d'insertion d'aiguille (6) comprenant un boîtier d'aiguille (30) possédant une embase d'aiguille (30c) séparant la section distale (30a) et la section proximale (30e) du boîtier d'aiguille (10), l'embase d'aiguille (30c) possédant, fixée à elle, une extrémité proximale (12e) d'une aiguille (12) possédant une pointe distale pointue (12a), l'aiguille (12) établissant un passage de fluide entre la pointe distale pointue (12a) et la section proximale (30e) du boîtier d'aiguille (30) ;
un protection de pince (34) ; et
une pince de sécurité (36) logée de manière non amovible dans la protection de pince (34) montée de manière coulissante le long de l'aiguille (12), la pince de sécurité (36) comprenant une base (36b) comportant deux bras (36c1, 36c2) s'étendant de manière distale à partir de la base (36b), les deux bras (36c1, 36c2) possédant des griffes distales respectives (36h, 36g) et des éléments respectifs de sollicitation naturelle vers l'intérieur (36d1b, 36d2b) ;
ladite aiguille (12) s'étendant à travers l'embase de cathéter (8) et sa pointe distale pointue (12a) s'étendant au-delà de l'extrémité distale (10b) du cathéter (10), ladite extrémité proximale (8d) de l'embase de cathéter (8) étant maintenue par les griffes distales (36h, 36g) des deux bras (36c1, 36c2) de la pince de sécurité (36) et de la protection de pince (34) entourée par la section distale (30a) du boîtier d'aiguille (30) lorsque l'ensemble cathéter se trouve dans une position prête.

2. Ensemble cathéter à système fermé selon la revendication 1, comprenant en outre :
un septum élastomère (16) ajusté à l'intérieur de l'embase de cathéter (8) à proximité de l'ouverture (8e) de l'orifice latéral (8f), le septum (16) étant adapté à être percé par l'aiguille (12) dans la position prête et se refermant lorsque l'aiguille est retirée du septum (16).

3. Ensemble cathéter à système fermé selon la revendication 1, comprenant en outre :
un dispositif de retenue de septum (22) inséré de manière non amovible dans l'extrémité proximale ouverte (8d) de l'embase de cathéter (8), le dispositif de retenue de septum (22) possédant une extrémité distale (22a) en butée avec l'extrémité proximale (16b) d'un septum (16) ajusté à l'intérieur de l'embase de cathéter (8) de manière à empêcher un déplacement proximal du septum (16).

4. Ensemble cathéter à système fermé selon la revendication 3, ledit septum (16) possédant une extrémité distale (16a) en contact non mobile avec une butée de paroi interne (8g) à proximité de l'ouverture (8e) de l'orifice latéral (8f) pour maintenir le septum (16) en place à l'intérieur de l'embase de cathéter (8) de sorte qu'après le retrait de l'aiguille (12) du cathéter (10), un trajet de communication de fluide soit établi entre le cathéter (10) et l'orifice latéral (8f) à travers la cavité interne (8b) de l'embase de cathéter (8).

5. Ensemble cathéter à système fermé selon la revendication 3, ladite extrémité distale (22a) du dispositif de retenue de septum (16) possédant une ouverture (22f) à travers laquelle passe l'aiguille (12) pour percer le septum (16) lorsque l'ensemble cathéter se trouve dans la position prête.

6. Ensemble cathéter à système fermé selon la revendication 1, ladite protection de pince (34) comprenant une section proximale (30d) conçue pour s'accoupler de manière ajustée à une ouverture distale (30c3) de l'embase d'aiguille (30c) et une chambre (34d) pour enfermer la pince de sécurité (36), la protection de pince (34) s'ajustant de manière amovible à la section distale (30a) du boîtier d'aiguille (30) dans la position prête.

7. Ensemble cathéter à système fermé selon la revendication 1, lesdits deux bras (36c1, 36c2) étant l'un et l'autre éléments allongés (36c1, 36c2) s'étendant chacun longitudinalement à partir de la base (36b) jusqu'à des coudes correspondants à l'endroit où les éléments allongés (36c1, 36c2) sont courbés de manière sensiblement orthogonale l'un vers l'autre pour former des parties courbées respectives possédant les griffes distales (36h, 36g), l'un parmi l'un et l'autre éléments allongés (36c1, 36c2) possédant une fente (36e4) à travers laquelle passe la partie courbée de l'autre parmi l'un et l'autre éléments allongés (36c1, 36c2), lesdits éléments respectifs de sollicitation naturelle vers l'intérieur (36d1b, 36d2b) étant pourvus au niveau de l'un et l'autre éléments allongés (36c1, 36c2) à proximité de leurs coudes correspondants de sorte que, lorsque l'ensemble cathéter se trouve dans la position prête, les éléments respectifs de sollicitation naturelle vers l'intérieur (36d1b, 36d2b) soient sollicités par l'aiguille passant entre eux pour solliciter les éléments allongés (36c1, 36c2) vers l'extérieur de manière à amener les parties courbées respectives à se déplacer l'une vers l'autre de sorte que l'extrémité proximale (8d) de l'embase de cathéter (8) soit saisie par les griffes distales (36h, 36g).

8. Ensemble cathéter fermé selon la revendication 1, ladite protection de pince (34) étant conçue pour être logée de manière ajustée dans la section distale (30a) du boîtier d'aiguille (10), la protection de pince (34) possédant une extrémité distale ouverte (34a) ; et
ladite pince de sécurité (36) étant enfermée de manière non amovible dans une chambre de la protection de pince (34) de sorte que les griffes distales (36h, 36g) de la pince de sécurité (36) soient enfermées par l'extrémité distale de la protection de pince (34).

9. Ensemble cathéter à système fermé selon la revendication 1, dans une position sûre, ladite pointe distale (12a) de l'aiguille (12) étant retirée de manière proximale au-delà des éléments de sollicitation (36d1b, 36d2b) de sorte que les éléments de sollicitation (36d1b, 36d2b) reviennent à leur position naturelle vers l'intérieur pour fournir une butée destinée à empêcher la pointe distale de l'aiguille de ressortir de la pince de sécurité (36).

10. Ensemble cathéter à système fermé selon la revendication 1, ladite base (36b) de la pince de sécurité (36) étant maintenue de manière fixe sur une partie proximale de la protection de pince (34) de sorte que la pince de sécurité (36) soit maintenue de manière non amovible à l'intérieur de la protection de pince (34), ladite protection de pince (34) étant mobile par rapport au boîtier d'aiguille (10).

11. Ensemble cathéter à système fermé selon la revendication 1, ladite section proximale (30e) du boîtier d'aiguille (10) étant une chambre de séparation avec un bouchon de séparation au niveau de l'ouverture proximale du boîtier d'aiguille (10) qui permet à l'air de passer mais empêche le fluide de sortir de la section proximale ; et
ladite embase de cathéter (8) possédant des ailes latérales (8i) s'étendant intégralement à partir de la paroi extérieure de l'embase de cathéter (8) et un tube d'extension (14) raccordé à l'orifice latéral.

12. Procédé de fabrication d'un ensemble cathéter de sécurité, comprenant les étapes de :
fourniture d'une embase de cathéter (8) possédant un corps comprenant une extrémité distale (8a), une extrémité proximale ouverte (8d) et une paroi (8c) s'étendant entre l'extrémité distale (8a) et l'extrémité proximale (8d) définissant une cavité interne (8b) ;
extension d'un cathéter (10) possédant une pointe distale (10a) à partir de l'extrémité distale (8a) de l'embase de cathéter (8) ;
fourniture d'un boîtier d'aiguille (10) possédant une section distale (30a), une section proximale (30e) et une embase d'aiguille (30c) prise en sandwich entre la section distale (30a) et la section proximale (30e),
fixation d'une aiguille (12) possédant une pointe distale pointue (12a) et une extrémité proximale (12e) à l'embase d'aiguille (30c) pour établir un passage de fluide entre la pointe distale pointue (12a) et la section proximale (30e) du boîtier d'aiguille (10) ;
positionnement amovible d'un protection de pince (34) dans la section distale (30a) du boîtier d'aiguille (10) ;
montage coulissant d'une pince de sécurité (36) le long de l'aiguille (12) ;
logement non amovible de la pince de sécurité (36) dans la protection de pince (34), la pince de sécurité (36) comprenant une base (36b) possédant deux bras (36c1, 36c2) s'étendant de manière distale à partir de la base (36b), les deux bras (36c1, 36c2) possédant des griffes distales respectives (36h, 36g) et des éléments respectifs de sollicitation naturelle vers l'intérieur (36d1b, 36d2b) ; et
extension de l'aiguille (12) à travers l'embase de cathéter (8) et sa pointe distale pointue (12a) au-delà de l'extrémité distale (10b) du cathéter (10) de sorte que l'extrémité proximale (8d) de l'embase de cathéter (8) soit maintenue de manière fixe par les griffes distales (36h, 36g) des deux bras (36c1, 36c2) de la pince de sécurité (36) et que la protection de pince (34) soit enfermée dans la section distale (30a) du boîtier d'aiguille (30) lorsque l'ensemble cathéter se trouve dans une position prête.

13. Procédé de fabrication d'un ensemble cathéter de sécurité selon la revendication 12, comprenant en outre les étapes de :
extension d'un orifice latéral (8f) à partir d'une ouverture (8e) au niveau de la paroi entre l'extrémité distale (8a) et l'extrémité proximale (8d) au niveau de l'embase de cathéter (8) ;
ajustement d'un septum élastomère (16) à l'intérieur de l'embase de cathéter (8) à proximité de l'ouverture (8e) de l'orifice latéral (8f), le septum (16) étant adapté à être percé par l'aiguille (12) dans la position prête et se refermant lorsque l'aiguille (12) est retirée du septum (16).

14. Procédé de fabrication d'un ensemble cathéter de sécurité selon la revendication 12, comprenant en outre les étapes de :
insertion non amovible d'un dispositif de retenue de septum (22) dans l'extrémité proximale ouverte (8d) de l'embase de cathéter (8) ;
mise en butée d'une extrémité distale (22a) du dispositif de retenue de septum (22) avec l'extrémité proximale (16b) d'un septum (16) ajusté à l'intérieur de l'embase de cathéter (8) de manière à empêcher un déplacement proximal du septum (16) ;
fourniture d'une butée de paroi interne (8g) à proximité de l'ouverture (8e) de l'orifice latéral (8f) pour empêcher le déplacement distal du septum (16) par rapport à l'embase de cathéter (8) de sorte que le septum (16) soit maintenu en place à l'intérieur de l'embase de cathéter (8).

15. Procédé de fabrication d'un ensemble cathéter de sécurité selon la revendication 12, comprenant en outre les étapes de :
configuration de la protection de pince (34) pour posséder une extension proximale (34c) pouvant s'accoupler de manière ajustée à une ouverture distale (30c3) de l'embase d'aiguille (30c) et une chambre (34d) pour enfermer la pince de sécurité (36), la protection de pince (34) s'ajustant à la section distale (30a) du boîtier d'aiguille (10) dans la position prête.
